# EUROPEAN PATENT APPLICATION

(11) **EP 2 823 766 A1**
(43) Date of publication of application: **14.01.2015**
(21) Application number: 14173047.3
(22) Date of filing: 18.06.2014
(51) Int. Cl.: A61B 8/08

(54) **Ultrasound system and method for providing object information**

(30) Priority: 04.07.2013 KR 20130078282
(71) Applicant: Samsung Medison Co., Ltd., Gangwon-do 250-870 (KR); Samsung Electronics Co., Ltd, Gyeonggi-do 443-742 (KR)
(72) Inventor: Ahn, Mi-jeoung, Gangwon-do (KR); Jin, Gil-ju, Gangwon-do (KR); Hyun, Dong-gyu, Gangwon-do (KR); Oh, Jung-taek, Seoul (KR); Jo, Jae-moon, Gyeonggi-do (KR)
(74) Representative: Schmid, Wolfgang

(57) **Abstract**

Disclosed are an ultrasound system and method for determining an object, for example, an artery or a vein, and provide object information corresponding to a position of the object. The ultrasound system includes: an ultrasound probe that transmits an ultrasound signal to a body comprising an object, and receives an ultrasound echo signal reflected from the body to generate a reception signal, an ultrasound data acquiring unit that acquires ultrasound data corresponding to the object in the body by using the reception signal, a processing unit that generates Doppler data by using the ultrasound data, analyzes the Doppler data to detect the object, and generates object information corresponding to a position of the detected object, and an object information providing unit that outputs the object information.

## Description

### RELATED APPLICATIONS

This application claims the benefit of Korean Patent Application No. 10-2013-0078282, filed on July 4, 2013, in the Korean Intellectual Property Office, the disclosure of which is incorporated herein in its entirety by reference.

### BACKGROUND

### 1. Field

One or more embodiments of the present invention relate to an ultrasound system, and more particularly, to an ultrasound system and method determining an object, for example, an artery and a vein, in order to provide object information corresponding to a position of the object.

### 2. Description of the Related Art

As an ultrasound system has noninvasive and nondestructive characteristics, it is widely used in medical applications for obtaining internal information of an object. The ultrasound system provides in real time a high-resolution image of an internal tissue of an object to a medical practitioner, without requiring a surgical operation to directly make an incision in the body to observe the object.

A method of inserting a needle into a vein to inject medication has been used for intensive treatment or application of continuous medication. In this method, medication is injected into a patient via a well know central vein access or peripheral vein access. In this case, a medical practitioner performs a landmark-based vein access based on anatomical knowledge without the help of an image, or finds a vein based on an ultrasound wave, X-ray, a computed tomography (CT), or magnetic resonance imaging (MRI).

When the vein access is used, arterial puncture, thrombus, and infection have been reported as the main side effects. In particular, when the vein access is performed by using an ultrasound image, a user unfamiliar with ultrasound images, like a surgeon or an anesthetist, may not distinguish an artery from a vein, thereby a medical accident such as artery damage or painful access being possible.

To prevent such a medical accident, the access is performed by using a transverse view that simultaneously shows an artery and a vein. However, in this case, an inserted needle or guide is not shown well in an ultrasound cross-sectional image.

To solve this problem, the following methods have been proposed. In the first method, a longitudinal view, in which a needle or a guide is well shown in an ultrasound cross-sectional image, is used along with a needle kit, and in the second method, the vein access is performed by using a transverse view that simultaneously shows an artery and a vein, position sensors are respectively attached to an ultrasound probe and a needle so as to locate the needle, and a display unit displays a relative position of the needle in an ultrasound cross-sectional image.

However, when a longitudinal view is used, an unskilled user may confuse an artery with a vein. Also, when the position sensor and the transverse view are use, space limitation occurs because an additional device such as a sensor is needed, a weight of an ultrasound probe increases, and the overall cost of the ultrasound system increases.

### SUMMARY

One or more embodiments of the present invention include an ultrasound system and method for detecting an internal object (i.e., an artery and a vein) of a human body by using Doppler data and providing object information corresponding to a position of the object.

One or more embodiments of the present invention include an ultrasound system and method for detecting an object (i.e., a blood vessel) by using Doppler data and accurately distinguishing an artery from a vein in the detected object in order to accurately provide a position of the object, thereby preventing a user from abnormally inserting a needle into the artery and moreover guiding the user in order to accurately insert the needle into the vein.

Additional aspects will be set forth in part in the description which follows and, in part, will be apparent from the description, or may be learned by practice of the presented embodiments.

According to one or more embodiments of the present invention, an ultrasound system includes: an ultrasound probe that transmits an ultrasound signal to a body comprising an object and receives an ultrasound echo signal reflected from the body to generate a reception signal; an ultrasound data acquiring unit that acquires ultrasound data corresponding to the object by using the reception signal; a processing unit that generates Doppler data by using the ultrasound data, analyzes the Doppler data to detect the object, and generates object information corresponding to a position of the detected object; and an object information providing unit that outputs the object information.

According to one or more embodiments of the present invention, an object information providing method includes: a) transmitting, by using an ultrasound probe, an ultrasound signal to a body comprising an object and receiving an ultrasound echo signal reflected from the body to generate a reception signal; b) acquiring ultrasound data corresponding to the object by using the reception signal; c) generating Doppler data by using the ultrasound data; d) analyzing the Doppler data to detect the object; e) generating object information corresponding to a position of the detected object; and f) outputting the object information.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram schematically illustrating a configuration of an ultrasound system according to an embodiment of the present invention;
FIG. 2 is a block diagram schematically illustrating a configuration of an ultrasound data acquiring unit according to an embodiment of the present invention;
FIG. 3 is a flowchart illustrating a method of determining an object to provide object information, according to an embodiment of the present invention;
FIG. 4 is an exemplary diagram illustrating an ultrasound probe, a transducer element, an ultrasound image, and an object according to an embodiment of the present invention;
FIG. 5 is an exemplary diagram illustrating an ultrasound probe, a light-emitting unit, and object information according to an embodiment of the present invention; and
FIG. 6 is an exemplary diagram illustrating an ultrasound probe, an image projector, and object information according to an embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

Hereinafter, embodiments of the present invention will be described in detail with reference to the accompanying drawings.

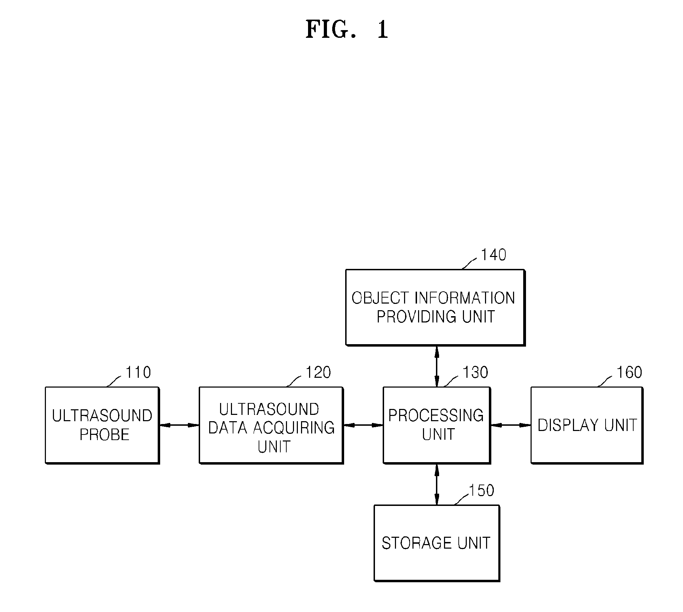

FIG. 1 is a block diagram schematically illustrating an ultrasound system 100 according to an embodiment of the present invention. Referring to FIG. 1, the ultrasound system 100 according to an embodiment of the present invention includes an ultrasound probe 110, an ultrasound data acquiring unit 120, a processing unit 130, an object information providing unit 140, a storage unit 150, and a display unit 160. Also, the ultrasound system 100 further includes a user input unit (not shown) for receiving input information of a user. The user input unit includes a control panel, a trackball, a touch screen, a keyboard, and a mouse.

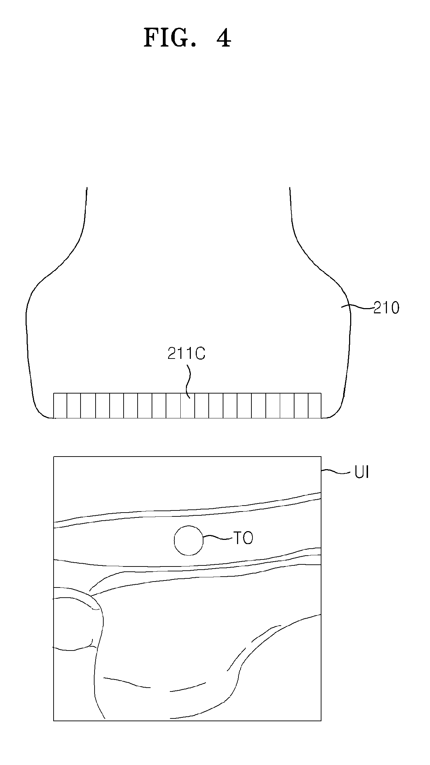

The ultrasound probe 110 includes a plurality of transducer elements 211 (see FIG. 4) that convert an electrical signal into an ultrasound signal. The ultrasound probe 110 transmits the ultrasound signal to a body. The body includes a plurality of objects (for example, arteries, veins, etc.). Also, the ultrasound probe 110 receives the ultrasound signal (i.e., an ultrasound echo signal) reflected from the body to generate an electrical signal (hereinafter referred to as a reception signal). The reception signal is an analog signal. The ultrasound probe 110 includes a convex probe and a linear probe.

In an embodiment, the ultrasound probe 110 transmits and receives the ultrasound signal while being in a fixed contact with a surface of the body. In another embodiment, the ultrasound probe 110 transmits and receives the ultrasound signal while moving in a certain direction in contact with the surface of the body.

The ultrasound data acquiring unit 120 controls transmission of the ultrasound signal. Also, the ultrasound data acquiring unit 120 acquires ultrasound data corresponding to an ultrasound image of the body by using the reception signal supplied from the ultrasound probe 110. The ultrasound data acquiring unit 120 may be implemented by using a processor that includes a central processing unit (CPU), a microprocessor, and a graphic processing unit (GPU).

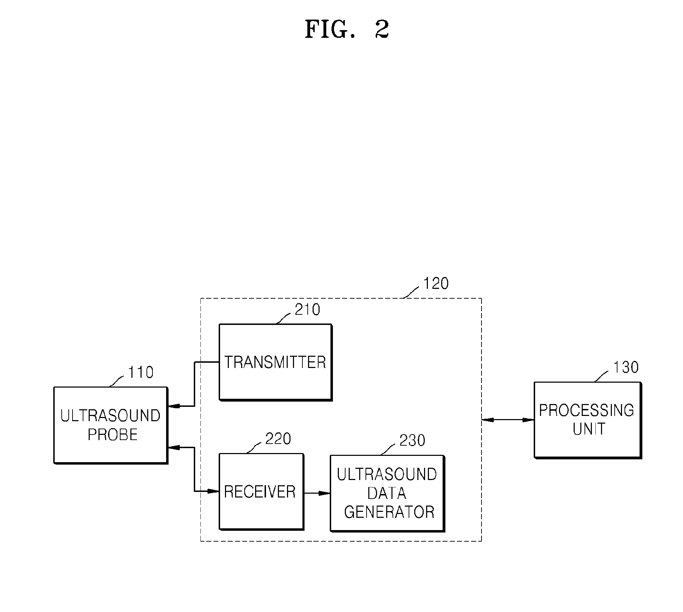

FIG. 2 is a block diagram schematically illustrating a configuration of the ultrasound data acquiring unit 120 according to an embodiment of the present invention. Referring to FIG. 2, the ultrasound data acquiring unit 120 includes a transmitter 210, a receiver 220, and an ultrasound data generator 230.

The transmitter 210 controls transmission of the ultrasound signal. Also, the transmitter 210 generates an electrical signal (hereinafter referred to as a transmission signal), which is used to obtain the ultrasound image, in consideration of the transducer element 211.

In an embodiment, the transmitter 210 generates a transmission signal (hereinafter referred to as a first transmission signal), which is used to obtain a first ultrasound image, in consideration of the transducer element 211. The first ultrasound image includes a brightness (B) mode image, but the present embodiment is not limited thereto. Therefore, when the ultrasound probe 110 is in fixed contact with the surface of the body, the ultrasound probe 110 converts the first transmission signal (supplied from the transmitter 210) into an ultrasound signal, transmits the converted ultrasound signal to the body, and receives an ultrasound echo signal reflected from the body to generate a reception signal (hereinafter referred to as a first reception signal).

Moreover, the transmitter 210 generates a transmission signal (hereinafter referred to as a second transmission signal), which is used to obtain a second ultrasound image, in consideration of the transducer element 211 and an ensemble number. The second ultrasound image includes a Doppler spectrum image, a color Doppler image, or a power Doppler image, but the present embodiment is not limited thereto. Therefore, when the ultrasound probe 110 is a fixed contact with the surface of the body, the ultrasound probe 110 converts the second transmission signal (supplied from the transmitter 210) into an ultrasound signal, transmits the converted ultrasound signal to the body, and receives an ultrasound echo signal reflected from the body to generate a reception signal (hereinafter referred to as a second reception signal).

In another embodiment, the transmitter 210 sequentially generates transmission signals (hereinafter referred to as third transmission signals), which are used to obtain a plurality of the first ultrasound images, in consideration of the transducer element 211. Therefore, while moving in a certain direction in contact with the surface of the body, the ultrasound probe 110 converts each of the third transmission signals (supplied from the transmitter 210) into an ultrasound signal, transmits the converted ultrasound signal to the body, and receives an ultrasound echo signal reflected from the body to generate a reception signal (hereinafter referred to as a third reception signal).

Moreover, the transmitter 210 sequentially generates transmission signals (hereinafter referred to as fourth transmission signals), which are used to obtain a plurality of the second ultrasound images, in consideration of the transducer element 211 and an ensemble number. Therefore, while moving in a certain direction in contact with the surface of the body, the ultrasound probe 110 converts each of the fourth transmission signals (supplied from the transmitter 210) into an ultrasound signal, transmits the converted ultrasound signal to the body, and receives an ultrasound echo signal reflected from the body to generate a reception signal (hereinafter referred to as a fourth reception signal).

The receiver 220 analog-digital converts the reception signal supplied from the ultrasound probe 110 to generate a digital signal. Also, the receiver 220 performs reception beamforming of the digital signal in consideration of the transducer element 211 to generate a reception focusing signal. The reception beamforming may be performed by various known methods, and thus, its detailed description is not provided in the present embodiment.

In an embodiment, the receiver 220 analog-digital converts the first reception signal supplied from the ultrasound probe 110 to generate a digital signal (hereinafter referred to as a first digital signal). The receiver 220 performs reception beamforming of the first digital signal in consideration of the transducer element 211 to generate a reception focusing signal (hereinafter referred to as a first reception focusing signal).

Moreover, the receiver 220 analog-digital converts the second reception signal supplied from the ultrasound probe 110 to generate a digital signal (hereinafter referred to as a second digital signal). The receiver 220 performs reception beamforming of the second digital signal in consideration of the transducer element 211 to generate a reception focusing signal (hereinafter referred to as a second reception focusing signal).

In another embodiment, the receiver 220 analog-digital converts the third reception signal supplied from the ultrasound probe 110 to generate a digital signal (hereinafter referred to as a third digital signal). The receiver 220 performs reception beamforming of the third digital signal in consideration of the transducer element 211 to generate a reception focusing signal (hereinafter referred to as a third reception focusing signal).

Moreover, the receiver 220 sequentially analog-digital converts the fourth reception signal supplied from the ultrasound probe 110 to generate a digital signal (hereinafter referred to as a fourth digital signal). The receiver 220 performs reception beamforming of the fourth digital signal in consideration of the transducer element 211 to generate a reception focusing signal (hereinafter referred to as a fourth reception focusing signal).

The ultrasound data generator 230 generates ultrasound data corresponding to an ultrasound image by using the reception focusing signal supplied from the receiver 220. Also, the ultrasound data generator 230 may perform various signal processing operations (for example, gain adjustment, etc.), which are necessary for generating the ultrasound data, on the reception focusing signal.

In an embodiment, the ultrasound data generator 230 generates ultrasound data (hereinafter referred to as first ultrasound data) corresponding to a first ultrasound image by using the first reception focusing signal supplied from the receiver 220. The first ultrasound data includes radio frequency (RF) data, but the present embodiment is not limited thereto.

Moreover, the ultrasound data generator 230 generates ultrasound data (hereinafter referred to as second ultrasound data) corresponding to a second ultrasound image by using the second reception focusing signal supplied from the receiver 220. The second ultrasound data includes in-phase/quadrature (I/Q) data, but the present embodiment is not limited thereto.

In another embodiment, the ultrasound data generator 230 generates ultrasound data (hereinafter referred to as third ultrasound data) corresponding to a third ultrasound image by using the third reception focusing signal supplied from the receiver 220.

Moreover, the ultrasound data generator 230 generates ultrasound data (hereinafter referred to as fourth ultrasound data) corresponding to a fourth ultrasound image by using the fourth reception focusing signal supplied from the receiver 220.

Referring again to FIG. 1, the processing unit 130 controls operations of the ultrasound probe 110, the ultrasound data acquiring unit 120, the object information providing unit 140, the storage unit 150, and the display unit 160. The processing unit 130 may be implemented by using a processor that includes a CPU, a microprocessor, and a GPU.

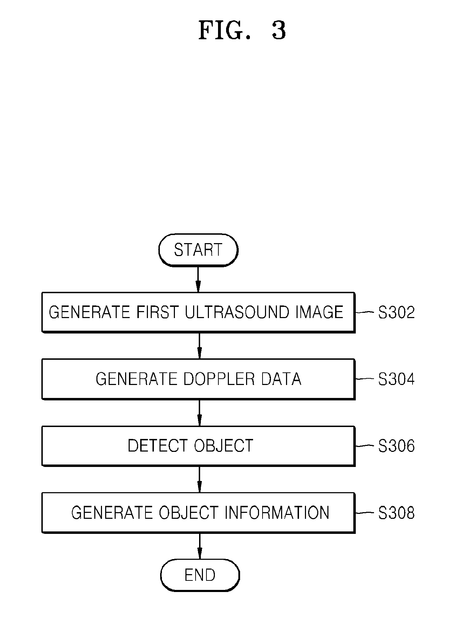

FIG. 3 is a flowchart illustrating a method of determining an object to provide object information, according to an embodiment of the present invention. Hereinafter, for convenience of description, an object is assumed to include a blood vessel (an artery or a vein) through which blood flows. Referring to FIG. 3, in operation S302, the processing unit 130 generates the first ultrasound image by using the ultrasound data (the first ultrasound data or the third ultrasound data) supplied from the ultrasound data acquiring unit 120.

In operation S304, the processing unit 130 generates Doppler data by using the ultrasound data supplied from the ultrasound data acquiring unit 120. The Doppler data indicates a velocity corresponding to a motion of an object, stiffness corresponding to the motion of the object, or a size of the object (i.e., a value indicating the blood flow). The Doppler data may be generated by various known methods, and thus, a detailed description thereof is not provided in the present embodiment.

In an embodiment, the processing unit 130 generates Doppler data corresponding to the second ultrasound image by using the second ultrasound data supplied from the ultrasound data acquiring unit 120. In another embodiment, the processing unit 130 generates Doppler data corresponding to a plurality of the second ultrasound images by using the fourth ultrasound data supplied from the ultrasound data acquiring unit 120.

In operation S306, the processing unit 130 detects an object by using the Doppler data. In the present embodiment, the processing unit 130 accumulates the Doppler data on a time axis. As an example, the processing unit 130 stores the Doppler data in the order of Doppler data input to a queue. As another example, the processing unit 130 summates the Doppler data to accumulate the Doppler data. The processing unit 130 calculates an average value of the accumulated Doppler data (a velocity, stiffness, or the amount of blood flow), and compares the calculated average value and a predetermined threshold value to detect the object. For example, the processing unit 130 calculates an absolute value of the calculated average value. The processing unit 130 compares a first predetermined threshold value (which is used to detect the object (in particular, the blood flow)) and the absolute value to detect an absolute value equal to or greater than the first predetermined threshold value. That is, the processing unit 130 detects the absolute value corresponding to the blood flow. The processing unit 130 compares a second predetermined threshold value (which is used to distinguish an artery and a vein) and the detected absolute value, and when it is determined that the detected absolute value is equal to or greater than the second predetermined threshold value, the processing unit 130 determines that an object corresponding to the detected absolute value is an artery (i.e., blood flow corresponding to the artery). On the other hand, when it is determined that the detected absolute value is less than the second predetermined threshold value, the processing unit 130 determines that an object corresponding to the detected absolute value is a vein (i.e., blood flow corresponding to the vein).

In operation S308, by using the detected object, the processing unit 130 generates information (hereinafter referred to as object information) corresponding to a position of the object.

In an embodiment, the processing unit 130 determines whether the detected object is located at a certain position with respect to the ultrasound probe 110, and when it is determined that the detected object is located at the certain position of the ultrasound probe 110, the processing unit 130 generates object information indicating the detected object located at the certain position of the ultrasound probe 110. In the present embodiment, the object information includes an alarm sound.

For example, as illustrated in FIG. 4, the processing unit 130 determines whether a detected object TO is located at a position corresponding to a transducer element 211 c disposed in a middle portion among the plurality of transducer elements 211 of the ultrasound probe 110, and generates object information according to the determination result. In FIG. 4, reference numeral UI refers to an ultrasound image (i.e., the first ultrasound image).

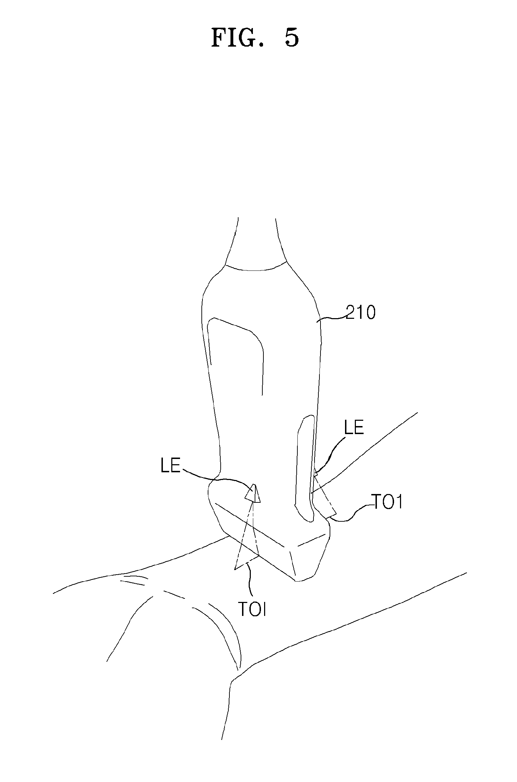

In another embodiment, the processing unit 130 determines whether the detected object is located at a certain position with respect to the ultrasound probe 110, and when it is determined that the detected object is located with respect to the certain position of the ultrasound probe 110, the processing unit 130 generates object information indicating the detected object being located at the certain position of the ultrasound probe 110. In the present embodiment, as illustrated in FIG. 5, the object information includes object information TOI for driving a light-emitting unit LE to display a position of the object via light.

In another embodiment, the processing unit 130 determines whether the detected object is located at a certain position with respect to the ultrasound probe 110, and when it is determined that the detected object is located at the certain position with respect to the ultrasound probe 110, the processing unit 130 generates object information indicating the detected object located at the certain position of the ultrasound probe 110. In the present embodiment, the object information includes object information for showing a position of the object via a vibration by driving a vibration unit (not shown) equipped in the ultrasound probe 110.

In another embodiment, the processing unit 130 performs image processing of the first ultrasound image on the basis of the detected object to extract an object image from the first ultrasound image, and generates object information including the extracted object image.

In another embodiment, the processing unit 130 performs image processing on each of a plurality of the first ultrasound images on the basis of an object detected from each of the plurality of second ultrasound images to extract an object image from each of the first ultrasound images, and generates object information including the extracted object image. That is, the processing unit 130 generates object information in which an object position is marked on a tissue part of the body through which the ultrasound probe 110 has passed.

In the above-described embodiments, the object information has been described to include an alarm, a driving signal, or an object image. However, the object information may include other various pieces of information.

Optionally, the processing unit 130 generates the second ultrasound image or the plurality of second ultrasound images by using the Doppler data. The second ultrasound image obtained from the Doppler data may be generated by various known methods, and thus, a detailed description thereof is not provided in the present embodiment.

Referring again to FIG. 1, the object information providing unit 140 provides (i.e., outputs) the object information generated by the processing unit 130 according to a control of the processing unit 130.

In an embodiment, the object information providing unit 140 includes a speaker (not shown). The speaker outputs object information (i.e., an alarm sound) according to a control of the processing unit 130. For example, the speaker is mounted on one side of the ultrasound probe 110. However, the speaker may be disposed at a position which enables a user to hear the alarm sound output therefrom.

In another embodiment, as illustrated in FIG. 5, the object information providing unit 140 includes the light-emitting unit LE. The light-emitting unit LE emits light according to a control of the processing unit 130 to output the object information TOI that shows a position of an object via light as illustrated in FIG. 5. The light-emitting unit LE, as illustrated in FIG. 5, is mounted on one side of the ultrasound probe 110.

In the above-described embodiments, two the light-emitting units LE have been described above as being mounted on the one side of the ultrasound probe 110. However, for example, at least one light-emitting unit may be mounted on the one side of the ultrasound probe 110.

In another embodiment, the object information providing unit 140 include a vibration unit (not shown). The vibration unit is driven according to a control of the processing unit 130 to output object information that shows a position of an object as vibration. The vibration unit is mounted on one side of the ultrasound probe 110.

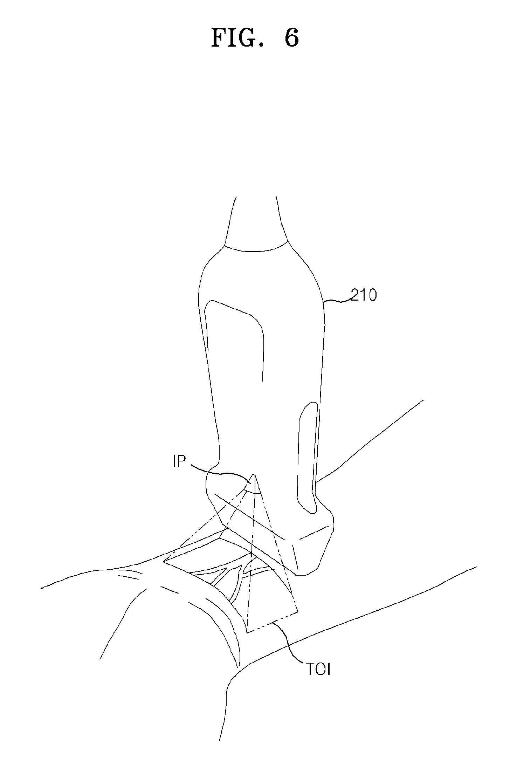

In another embodiment, as illustrated in FIG. 6, the object information providing unit 140 includes an image projector IP. As an example, the image projector IP is driven according to a control of the processing unit 130 to output object information including an object image. The image projector IP, as illustrated in FIG. 6, is mounted on one side of the ultrasound probe 110, and outputs object information about a surface of a body. As another example, the image projector IP is driven according to a control of the processing unit 130 to output object information corresponding to a position of an object and a movement and position (i.e., a position through which the ultrasound probe 110 has passed) of the ultrasound probe 110.

Referring again to FIG. 1, the storage unit 150 stores ultrasound data (the first and second ultrasound data) acquired by the ultrasound data acquiring unit 120. Also, the storage unit 150 stores object information generated by the processing unit 130. The storage unit includes a hard disk, a nonvolatile memory, a compact disc-read only memory (CD-ROM), and a digital versatile disc-read only memory (DVD-ROM).

The display unit 160 displays the first ultrasound image(s) generated by the processing unit 130. Also, the display unit 160 displays the second ultrasound image(s) generated by the processing unit 130. The display unit 160 includes a liquid crystal display (LCD), a light-emitting diode (LED) display, an organic light-emitting diode (OLED) display, or the like.

In the above-described embodiments, the ultrasound data acquiring unit 120 and the processing unit 130 have been described above as being different processors. However, in another embodiment, the ultrasound data acquiring unit 120 and the processing unit 130 may be implemented as one processor.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. An ultrasound system comprising:
an ultrasound probe that transmits an ultrasound signal to a body comprising an object and receives an ultrasound echo signal reflected from the body to generate a reception signal;
an ultrasound data acquiring unit that acquires ultrasound data corresponding to the object by using the reception signal;
a processing unit that generates Doppler data by using the ultrasound data, analyzes the Doppler data to detect the object, and generates object information corresponding to a position of the detected object; and
an object information providing unit that outputs the object information.

2. The ultrasound system of claim 1, wherein the object is at least one of an artery and a vein.

3. The ultrasound system of claim 2, wherein the Doppler data comprises at least one of a velocity corresponding to a motion of the object, stiffness corresponding to the motion of the object, and a size of the object.

4. The ultrasound system of claim 3, wherein the processing unit accumulates the Doppler data on a time axis, calculates an average value of the accumulated Doppler data, and compares the calculated average value and a predetermined threshold value to detect the object.

5. The ultrasound system of claim 4, wherein the processing unit calculates an absolute value of the calculated average value, compares a first predetermined threshold value used to detect the object and the absolute value to detect an absolute value equal to or greater than the first predetermined threshold value, and compares a second predetermined threshold value, used to distinguish the artery from the vein, and the detected absolute value to set an object corresponding to the detected absolute value as the artery or the vein.

6. The ultrasound system of claim 1, wherein the processing unit determines whether the detected object is located at a certain position with respect to the ultrasound probe in order to generate object information indicating the detected object that is located at the certain position with respect to the ultrasound probe.

7. The ultrasound system of claim 6, wherein the object information providing unit comprises a speaker that outputs the object information using an alarm sound.

8. The ultrasound system of claim 6, wherein the object information providing unit comprises a light-emitting unit that outputs the object information using a light.

9. The ultrasound system of claim 6, wherein the object information providing unit comprises a vibration unit that outputs the object information using a vibration.

10. The ultrasound system of claim 1, wherein the processing unit extracts, based on the position of the detected object, an object image corresponding to the object from an ultrasound image, and generates the object information including the object image.

11. The ultrasound system of claim 10, wherein the object information providing unit comprises an image projector that projects the object image.

12. The ultrasound system of claim 11, wherein,
the ultrasound probe performs a plurality of times an operation to transmit an ultrasound signal to the body while moving in a certain direction, and receives an ultrasound echo signal reflected from the body in order to generate a reception signal,
the ultrasound data acquiring unit acquires ultrasound data corresponding to each of a plurality of ultrasound images by using the reception signal,
the processing unit generates a plurality of ultrasound images by using the ultrasound data, detects the object by using each of the plurality of ultrasound images, performs image processing on each of the plurality of ultrasound images based on the detected object in order to extract an object image corresponding to the object in order to generate object information including a position of the object in the body through which the ultrasound probe has passed, and
the object information providing unit provides the object information.

13. An object information providing method comprising:
a) transmitting, by using an ultrasound probe, an ultrasound signal to a body comprising an object and receiving an ultrasound echo signal reflected from the body in order to generate a reception signal;
b) acquiring ultrasound data corresponding to the object by using the reception signal;
c) generating Doppler data by using the ultrasound data;
d) analyzing the Doppler data in order to detect the object;
e) generating object information corresponding to a position of the detected object; and
f) outputting the object information.

14. The object information providing method of claim 13, wherein step e) comprises determining whether the detected object is located at a certain position with respect to the ultrasound probe in order to generate object information indicating the detected object that is located at the certain position with respect to the ultrasound probe, and wherein step f) comprises outputting, by the object information providing unit, a line as the object information by using light.

15. The object information providing method of claim 13, wherein step e) comprises:
extracting, based on the position of the detected object, an object image corresponding to the object from an ultrasound image; and
generating the object information including the object image, wherein step f) comprises projecting, by the object information providing unit, the object image.
